(19) Europäisches Patentamt / European Patent Office / Office européen des brevets

(11) **EP 3 570 738 B1**

(12) **EUROPEAN PATENT SPECIFICATION**

(45) Date of publication and mention
of the grant of the patent:
**24.04.2024 Bulletin 2024/17**

(21) Application number: **18741954.4**

(22) Date of filing: **18.01.2018**

(51) International Patent Classification (IPC):
***A61B 5/024*** *(2006.01)*      ***A61B 5/00*** *(2006.01)*

(52) Cooperative Patent Classification (CPC):
**A61B 5/0261; A61B 5/02125; A61B 5/0816;
A61B 5/14551; A61B 5/6819;** A61B 5/7278

(86) International application number:
**PCT/US2018/014280**

(87) International publication number:
**WO 2018/136662 (26.07.2018 Gazette 2018/30)**

(54) **PULSE OXIMETRY SENSOR**

PULSOXIMETRIESENSOR

CAPTEURS D'OXYMÉTRIE DE POULS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.01.2017 US 201715410684**

(43) Date of publication of application:
**27.11.2019 Bulletin 2019/48**

(73) Proprietor: **General Electric Company
Schenectady, NY 12345 (US)**

(72) Inventors:
• **HUIKU, Matti Veli Tapani**
**00510 Helsinki (FI)**
• **RUPONEN, Pellervo**
**00510 Helsinki (FI)**
• **LAMMINMAKI, Sakari Matias**
**00510 Helsinki (FI)**

(74) Representative: **Fennell, Gareth Charles
Kilburn & Strode LLP
Lacon London
84 Theobalds Road
London WC1X 8NL (GB)**

(56) References cited:
WO-A1-2016/067153     US-A1- 2010 210 926
US-A1- 2015 038 810     US-A1- 2015 216 479
US-A1- 2016 291 345     US-B1- 7 648 463

• **CONSTANT NICHOLAS ET AL: "Pulse-Glasses:
An unobtrusive, wearable HR monitor with
Internet-of-Things functionality", 2015 IEEE 12TH
INTERNATIONAL CONFERENCE ON WEARABLE
AND IMPLANTABLE BODY SENSOR NETWORKS
(BSN), IEEE, 9 June 2015 (2015-06-09), pages 1-5,
XP032795016, DOI: 10.1109/BSN.2015.7299350
[retrieved on 2015-10-15]**

**Description**

FIELD

Embodiments of the subject matter disclosed herein relate to biological probes, sensors, and methods, and in particular, to photoplethysmography probes, sensors, and methods.

BACKGROUND

Photoplethysmography (PPG) relates to the use of optical signals transmitted through or reflected by blood-perfused tissues for monitoring a physiological parameter of a subject (also referred to as a patient herein). In this technique, one or more emitters are used to direct light at a tissue, and one or more detectors are used to detect the light that is transmitted through or reflected by the tissue. The volume of blood of the tissue affects the amount of light that is transmitted or reflected, which is output as a PPG signal. As the blood volume in a tissue changes with each heartbeat, the PPG signal also varies with each heartbeat.

Pulse oximetry is, at present, the standard of care for continuously monitoring arterial oxygen saturation ($SpO_2$). Pulse oximeters include PPG probes that use red (-660 nm) and infrared (-940 nm) light to determine physiological parameters (e.g., blood characteristics) of the subject, including $SpO_2$, pulse rate, and pulsating blood flow (e.g., blood perfusion) at the site of measurement. Conventional pulse oximetry probes are typically mounted to an extremity of the subject (e.g., a finger or ear lobe). However, during severe physiological stress, such as hypotension (low blood pressure), hypothermia (low body temperature), and volumetric shock (low blood volume), the body responds by constricting blood vessels (vasoconstriction) in order to divert blood away from the extremities and the periphery to maximize blood flow to central, vital organs (e.g., the brain, heart, and liver). Vasoconstriction, which may be regulated by the autonomic nervous system (ANS), may result in insufficient pulse amplitude for a pulse oximeter to reliably measure blood or blood circulation characteristics.

US 7648463 B1 describes a monitoring device and a method for monitoring the health of a user. The monitoring device preferably includes eyewear with an optical sensor, a digital storage and processing device with a display member and a control component, and a connection cable. The monitoring device preferably displays the following information about the user: pulse rate; calories expended by the user of a pre-set time period; target zones of activity; time; and distance traveled. US2015038810 A1 is directed to methods for monitoring an individual including securing a photoplethysmography (PPG) sensor comprising at least one emitter and at least one detector onto skin overlying an ophthalmic artery region of the individual; and obtaining PPG signals generated by the PPG sensor. Sensors for photoplethysmographic monitoring at the ophthalmic artery region are also provided.

N. Constant, O. Douglas-Prawl, S. Johnson and K. Mankodiya, "Pulse-Glasses: An unobtrusive, wearable HR monitor with Internet-of-Things functionality," 2015 IEEE 12th International Conference on Wearable and Implantable Body Sensor Networks (BSN), Cambridge, MA, USA, 2015, pp. 1-5 describes Pulse-Glasses that are cloud-connected, wearable, smart eyeglasses for unobtrusive and continuous heart rate (HR) monitoring. N. Constant et al 3D-printed the first prototype of Pulse-Glasses that use a photoplethysmography (PPG) sensor on one of the nose-pads to collect HR data. N. Constant et al integrated other circuits including an embedded board with Bluetooth low energy (BLE) and a rechargeable battery inside the two temples of Pulse-Glasses. N. Constant et al implemented IoT functionalities such that HR data are recorded from Pulse-Glasses, visualized on an Android smartphone, and stored seamlessly on the cloud. N. Constant et al present the developments of Pulse-Glasses hardware including IoT services and the preliminary results from validation experiments. N. Constant et al compared Pulse-Glasses with a laboratory ECG system to cross-validate HR data collected during various activities-sitting, talking, and walking-performed by a participant. N. Constant et al used Pulse-Glasses to record HR data of a driver to test IoT functionalities of location services and BLE and cloud connectivity.

BRIEF DESCRIPTION

In one embodiment, a system for an optical probe comprises a light emitter and a light detector coupled to a substrate, the light emitter and light detector configured to measure blood originating at least partly from an internal carotid artery of a patient; and an attachment mechanism configured to couple the optical probe to a nose of the patient, the light emitter and light detector positioned to be on opposite sides of the nose of the patient at a root of a nasal bridge when the optical probe is worn by the patient.

Thus, blood from the internal carotid artery, which does not undergo substantial ANS regulation, may be monitored, resulting in strong PPG signals even under physiological stress conditions such as hypothermia and hypovolemia.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description.

BRIEF DESCRIPTION OF THE DRAWINGS

The present invention will be better understood from reading the following description of non-limiting embodiments, with reference to the attached drawings, wherein below:

FIG. 1 is a block diagram illustrating one embodiment of a multi-wavelength pulse oximetry system.

FIG. 2A shows a cutaway side view of an example nasal pulse oximetry probe.

FIG. 2B shows a top view of the example nasal pulse oximetry probe.

FIG. 3A is a partial profile view of a human face illustrating how a nasal pulse oximetry probe may be positioned on a nose with respect to internal vasculature of the nose.

FIG. 3B is a partial profile view of a human face illustrating how a nasal pulse oximetry probe may be positioned on a nose with respect to other anatomical features of the face.

FIG. 4 is a partial front view of a face of a subject wearing a first embodiment of a nasal pulse oximetry probe.

FIG. 5 is a partial front view of a face of a subject wearing a second embodiment of a nasal pulse oximetry probe.

FIG. 6 is a high-level method for calculating physiological parameters from a PPG signal measured by a nasal pulse oximetry sensor.

FIG. 7 shows an example method for calculating $SpO_2$ of a patient based on the PPG signal data obtained by a nasal pulse oximetry sensor.

FIG. 8 shows an example method for calculating pulse transit time (PTT) of a patient from PPG signal data obtained by a nasal pulse oximetry sensor.

FIG. 9 shows an example method for calculating respiration rate of a patient from PPG signal data obtained by a nasal pulse oximetry sensor.

FIG. 10 shows an example method for determining if hypovolemia is indicated from PPG signal data obtained by a nasal pulse oximetry sensor.

FIG. 11 shows an example method for determining blood circulation at a measurement site from PPG signal data obtained by a nasal pulse oximetry sensor.

FIG. 12 is an example graph showing how separate $SpO_2$ values may be determined for systole and diastole.

FIG. 13 is an example graph showing microchanges of the saturation of arterial blood within one cardiac cycle plotted against heartbeat count.

FIG. 14 is an example graph showing fast Fourier transformed PPG signal data for a normovolemic subject and a hypovolemic subject.

FIG. 15 is an example graph showing the effect of different amounts of positive end-expiratory pressure on PPG signal data obtained by a nasal pulse oximetry sensor.

## DETAILED DESCRIPTION

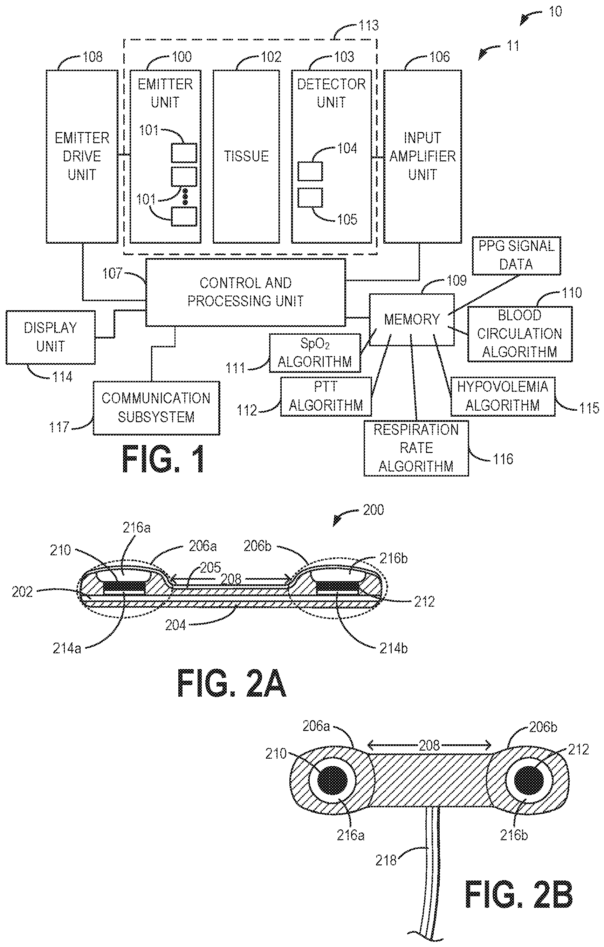

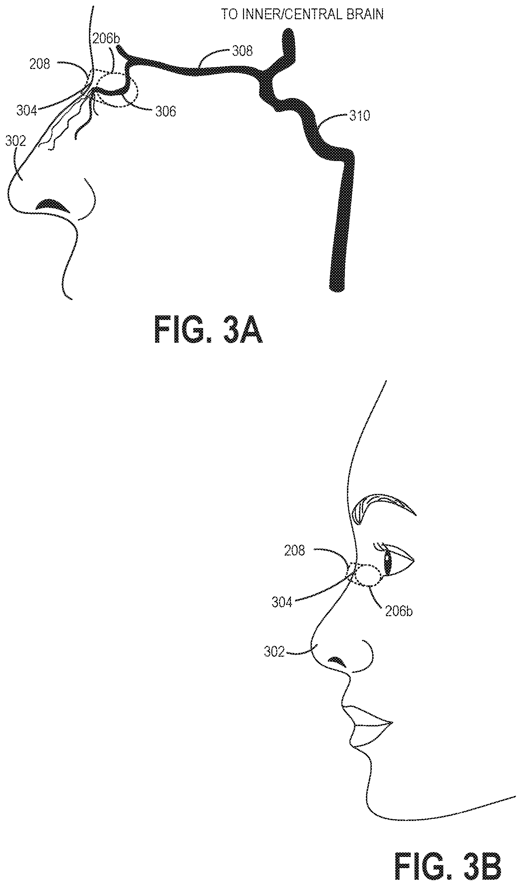

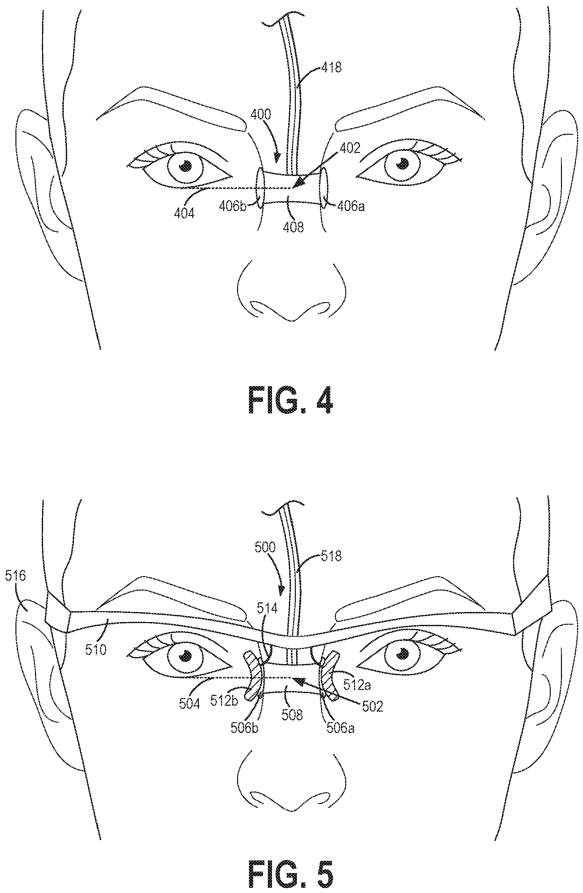

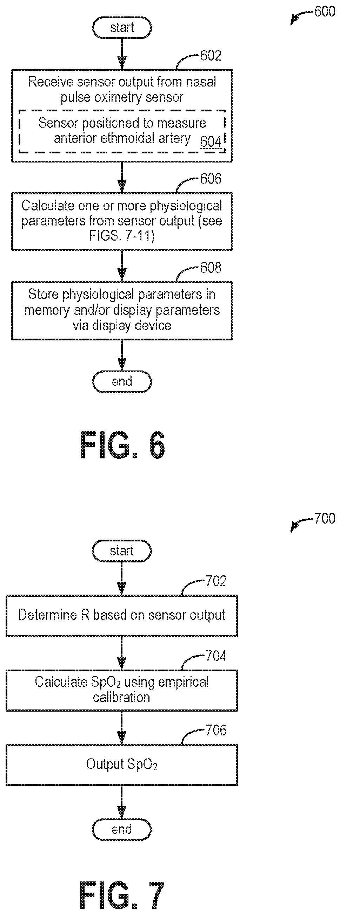

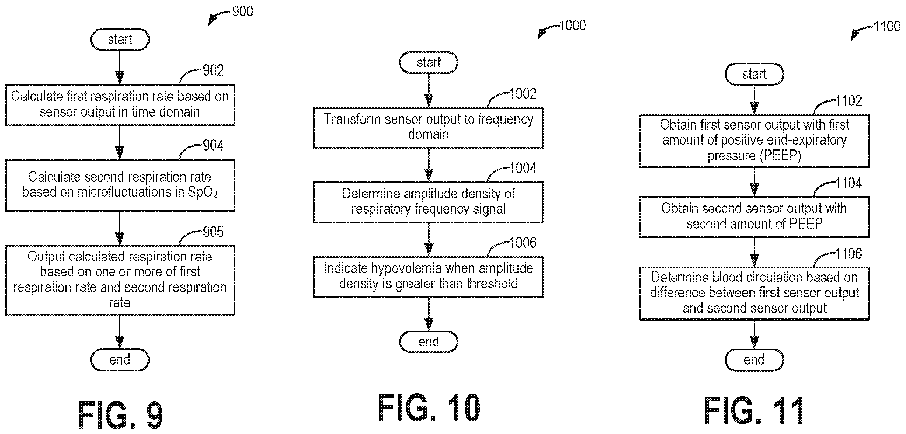

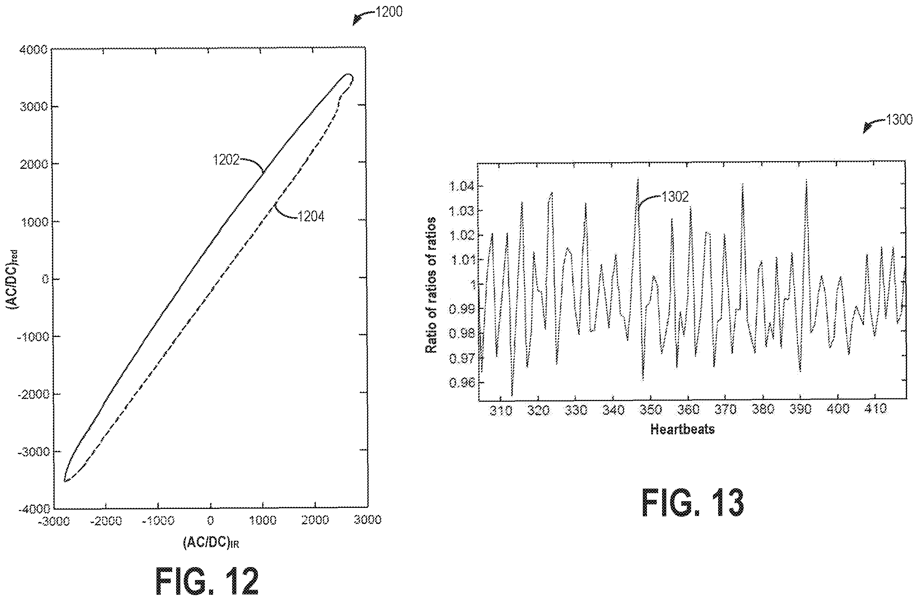

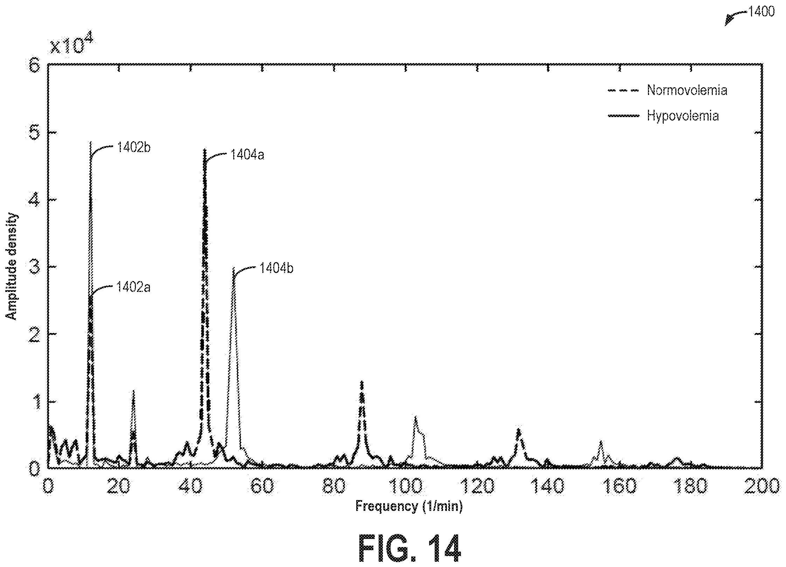

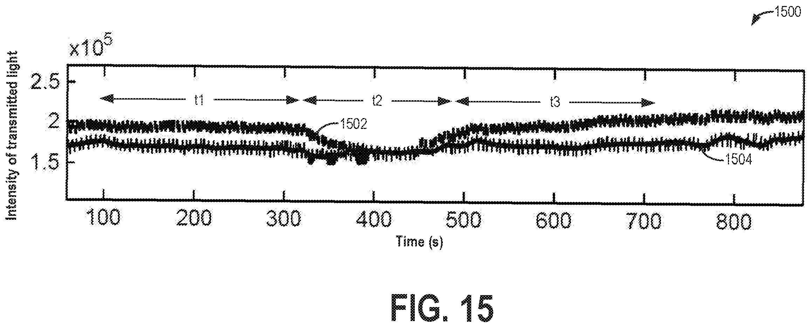

The following description relates to various embodiments of an optical probe, such as the nasal pulse oximetry probe shown in FIGS. 2A and 2B. The probe may be included in a pulse oximetry sensor or system, such as the system of FIG. 1, for determining physiological parameters of a patient. The probe is configured to be positioned on a root of a nasal bridge of the patient, as illustrated in FIGS. 3A-3B, to measure blood of an anterior ethmoidal artery. According to one embodiment of the invention, the probe may be attached to the patient without an external pressure element, as shown in FIG. 4. According to another embodiment of the invention, the probe may be attached to the patient with an added pressure element, as shown in FIG. 5. Output of the probe (e.g., a PPG waveform) may be used to calculate physiological parameters, for example, using the methods of FIGS. 6-11. In particular, respiration rate may be determined in more than one way due to strong respiratory-related variation measured in blood of the anterior ethmoidal artery, as described in the method of FIG. 9 and illustrated in the graphs of FIGS. 12-13. Further, the graph of FIG. 14 illustrates how the respiratory-related variation may be used to determine hypovolemia in the patient, as also described in the method of FIG. 10. The graph of FIG. 15 illustrates an example of the effect of positive end-expiratory pressure (PEEP) on the PPG waveform, which may be used to determine blood circulation, for example, according to the method of FIG. 11.

A pulse oximeter comprises a computerized measuring unit and a probe attached to a patient, typically a finger or ear lobe of the patient. The probe includes a light source for sending an optical signal through tissue of the patient and a photo detector for receiving the signal transmitted through or reflected from the tissue. On the basis of the transmitted and received signals, light absorption by the tissue may be determined. During each cardiac cycle, light absorption by the tissue varies cyclically. During the diastolic phase, absorption is caused by venous blood, non-pulsating arterial blood, cells and fluids in tissue, bone, and pigments. The level of light transmitted at end of the diastolic phase is typically referred to as the "DC component" of the total light transmission. During the systolic phase, there is an increase in light absorption (e.g., a decrease in transmitted light) compared with the diastolic phase due to the inflow of arterial blood into the tissue on which the pulse oximetry probe is attached. A crucial pulse oximetry principle is how the measurement can be focused on the blood volume representing the arterial blood only. In pulse oximetry, this is done by taking the pulsating arterial blood portion (the "AC signal") from the total transmission signal and normalizing this signal by the "DC" component. The resulting "AC/DC" signal is called the PPG waveform. Pulse oximetry is thus based on the assumption that the pulsatile component of the absorbance is due to arterial blood only.

In pulse oximetry, arterial blood is typically modeled as containing two species of hemoglobin: oxyhemoglobin (HbO$_2$) and reduced hemoglobin (Hb). Oxyhemoglobin is hemoglobin that is fully saturated with oxygen, and reduced hemoglobin is without oxygen. Arterial oxygen saturation measured by pulse oximetry (SpO$_2$) is defined as the percentage of HbO$_2$ divided by the total amount of hemoglobin (HbO$_2$ + Hb). In order to distinguish between the two species of hemoglobin, light absorption is measured at two different wavelengths. The probe of a traditional pulse oximeter includes two different light sources, such as light-emitting diodes (LEDs) or lasers, that emit light at two different wavelengths. The wavelength values widely used are 660 nm (red light) and 900 nm (infrared light), as the two species of hemoglobin have substantially different absorption at these wavelengths. Each light source is illuminated in turn at a frequency that is typically several hundred Hz.

FIG. 1 is a block diagram of one embodiment of a multi-wavelength pulse oximetry system 10. Light transmitted from an emitter unit 100 passes into patient tissue 102. The emitter unit includes multiple light sources 101, such as light-emitting diodes (LEDs), with each light source having a dedicated wavelength. Each wavelength forms one measurement channel on which PPG waveform data are acquired. The number of sources/wavelengths is at least two.

The light transmitted through the tissue 102 is received by a detector unit 103, which comprises two photo detectors 104 and 105 in this example. For example, photo detector 104 may be a silicon photodiode, and photo detector 105 may be a second silicon photodiode with different spectral characteristics or an indium gallium arsenide (InGaAs) photodiode. The emitter and detector units form a probe detector subunit 113 of the pulse oximetry system 10. The photo detectors convert the optical signals received into electrical pulse trains and feed them to an input amplifier unit 106. The amplified measurement channel signals are further supplied to a control and processing unit 107, which executes instructions stored in memory to convert the signals into digitized format for each wavelength channel.

The control and processing unit 107 further controls an emitter drive unit 108 to alternately activate the light sources. As mentioned above, each light source is typically illuminated several hundred times per second. With each light source being illuminated at such a high rate compared to the pulse rate of the patient, the control and processing unit 107 obtains a high number of samples at each wavelength for each cardiac cycle of the patient. The value of these samples varies according to the cardiac cycle of the patient, the variation being caused by the arterial blood.

The input amplifier unit 106, the control and processing unit 107, the emitter drive unit 108, and probe detector subunit 113 collectively form a probe 11. As used herein, the term "probe" may refer to the probe 11 and the attachment parts that attach the optical components, the probe 11, to the tissue site. The term "SpO$_2$ sensor" may refer to a unit comprising a probe, an analog front end, and a signal processing unit that calculates SpO$_2$ and other blood characteristics. In a multi-parameter body area network system, the system typically represents a set of multiple sensors, e.g., the different physiological parameter measurements. Therefore, the whole measurement system may comprise of several sensors and their associated probes, and the sensors may communicate to a common hub in which the parameters' information is integrated.

The digitized PPG signal data at each wavelength may be stored in a memory 109 of the control and processing unit 107 before being processed further according to non-transitory instructions (e.g., algorithms) executable by the control and processing unit 107 to obtain physiological parameters. For example, memory 109 may comprise a suitable data storage medium, for example, a permanent storage medium, removable storage medium, and the like. Additionally, memory 109 may be a non-transitory storage medium. In some examples, the system 10 may include a communication subsystem 117 operatively coupled to one or more remote computing devices, such as hospital workstations, smartphones, and the like. The communication subsystem 117 may enable the output from the detector units (e.g., the digitized PPG signal data) to be sent to the one or more remote computing devices for further processing and/or the communication subsystem may enable the output from the algorithms discussed below (e.g., determined physiological parameters) to be sent to the remote computing devices. The communication subsystem 117 may include wired and/or wireless communication devices compatible with one or more different communication protocols. As non-limiting examples, the communication subsystem 117 may be configured for communication via a wireless telephone network, a local- or wide-area network, and/or the Internet.

Algorithms may utilize the same digitized signal data and/or results derived from the algorithms and stored in the memory 109, for example. For example, for the determination of oxygen saturation and pulse transit time (PTT), the control and processing unit 107 is adapted to execute an SpO$_2$ algorithm 111 and a PTT algorithm 112, respectively, which may also be stored in the memory 109 of the control and processing unit 107. Further, a blood pressure algorithm 110, a hypovolemia algorithm 115, and a respiration rate algorithm 116 may also be stored in the memory 109 for determining blood pressure, an indication of hypovolemia, and respiration rate, respectively. The use of such algorithms will be described in more detail below with respect to FIGS. 6-11. The obtained physiological parameters and waveforms may be shown on a screen of a display unit 114. Further, in some examples, the control and processing unit, memory, and/or other subsystems may be located remotely from the rest of the sensor on a separate device, and the signal data from the detector units may be sent to the separate device for processing.

As used herein, the terms "sensor," "system," "unit," or "module" may include a hardware and/or software system that operates to perform one or more functions. For example, a sensor, module, unit, or system may include a computer processor, controller, or other logic-based device that performs operations based on instructions stored on a tangible and non-transitory computer readable storage medium, such as a computer memory. Alternatively, a sensor, module, unit, or system may include a hard-wired device that performs operations based on hard-wired logic of the device. Various modules or units shown in the attached figures may represent the hardware that operates based on software or hardwired instructions, the software that directs hardware to perform the operations, or a combination thereof.

"Systems," "units," "sensors," or "modules" may include or represent hardware and associated instructions (e.g., software stored on a tangible and non-transitory computer readable storage medium, such as a computer hard drive, ROM, RAM, or the like) that perform one or more operations described herein. The hardware may include electronic circuits that include and/or are connected to one or more logic-based devices, such as microprocessors, processors, controllers, or the like. These devices may be off-the-shelf devices that are appropriately programmed or instructed to perform operations described herein from the instructions described above. Additionally or alternatively, one or more of these devices may be hard-wired with logic circuits to perform these operations.

FIGS. 2A and 2B show diagrams of an example probe 200 of a pulse oximetry system (e.g., pulse oximetry system 10) that may be used to measure blood parameters at a nasal site. Specifically, probe 200 may be affixed to a nasal bridge of a patient, as will be described below with regard to FIGS. 3-5. FIG. 2A is a cutaway side view of probe 200, and FIG. 2B is a top view.

As shown in FIG. 2A, probe 200 may include a flexible substrate 202 that may give the probe rigidity while conforming to the nasal anatomy of the patient. For example, the thickness of flexible substrate 202 may be such that probe 200 can bend around and maintain contact with the nose of the patient. The flexible substrate 202 may be enveloped in a soft, cushion-like sleeve 204. For example, sleeve 204 may be comprised of a compressible foam padding wrapped in an opaque, flexible material, such as vinyl. The opaque material may both help prevent light from escaping the probe and help prevent ambient light from being introduced into the probe. The top portion of probe 200 may be covered with an adhesive 205 to enable attachment of the probe to the patient. For example, adhesive 205 may be a transparent, porous, flexible tape.

Probe 200 may include two cushion portions 206a and 206b, one on each end of the length of the probe and connected by a bridge portion 208. As shown, cushion portions 206a and 206b have a thicker amount of sleeve 204 than bridge portion 208 and are somewhat convex. In some examples, cushion portions 206a and 206b may have a more square shape, and in other examples, cushion portions 206a and 206b may have a more rounded shape. Cushion portion 206a houses an emitter unit 210, and cushion portion 206b houses a detector unit 212. In other examples, cushion portion 206a houses the detector unit 212 and cushion portion 206b houses the emitter unit 210. As described with reference to FIG. 1, emitter unit 210 may include multiple light sources, such as LEDs. Similarly, detector unit 212 may include multiple photo detectors. Emitter unit 210 and detector unit 212 may be fastened to flexible substrate 202 with adhesives 214a and 214b, respectively. Adhesives 214a and 214b may be an electrically conductive epoxy adhesive, for example. Cushion portion 206a may include a transparent portion 216a at the position of the emitter unit 210 such that light from emitter unit 210 may pass through cushion portion 206a. Similarly, cushion portion 206b may include a transparent portion 216b at the position of the detector unit 212 such that light may pass through cushion portion 206b to detector unit 212. For example, transparent portions 216a and 216b may be comprised of a transparent, deformable gel. An advantage of the deformable gel is that it may mold to sides of the nasal bridge and act to distribute pressure over a larger area relative to other transparent mediums.

As described with reference to FIG. 1, the probe 200 may be in communication with other components of the pulse oximetry system, such as an emitter drive unit (e.g., emitter drive unit 108), an input amplifier unit (e.g., input amplifier unit 106), a control and processing unit (e.g., control and processing unit 107), a memory (e.g., memory 109), and a display unit (e.g., display unit 114). As shown in FIG. 2B, probe 200 may include electric wirings 218 to communicate with the other components of the pulse oximetry system.

As also shown in FIG. 2B, probe 200 has a T-shape structure, with opposite T-ends housing the emitter unit 210 and the detector unit 212 and the electric wirings 218 comprising the T-foot. However, it should be noted that the size and shape of probe 200 may vary. In some examples, a single size and shape of probe may be used on all patients. In other examples, the probe may be manufactured at different sizes and configurations to accommodate different types of patients (e.g., neonates, infants, pediatrics, and adults) and to accommodate different nose sizes and shapes.

FIGS. 3A-3B are diagrams illustrating how the example probe 200 of FIG. 2 may be positioned on a patient in order to obtain PPG signals from blood originating from the internal carotid artery. FIG. 3A is a partial profile view of a human face illustrating how the probe may be positioned on a nose with respect to internal vasculature. FIG. 3B shows a second partial profile view of a human face illustrating the position of the probe in the context of other anatomical features of the face, such as the eyes. As shown in FIG. 3A and 3B, probe 200 is shaped to be positioned on nose 302 at the root of the nasal bridge 304. As used herein, the nasal bridge may include a saddle-shaped area of the nose that includes the nasal root and the lateral aspects of the nose and may lie between the glabella and the inferior boundary of the nasal bone and extend laterally to the inner canthi. The root of the nasal bridge may include an area of the nose that is the most depressed, superior part of the nose along the nasal ridge. The bridge portion 208 of probe 200 is bent over the root of the nasal bridge 304 such that one cushion portion is positioned on each side of nose 302. While only one cushion portion 206b is illustrated, it can be assumed that cushion portion 206a is positioned on the opposite side of nose 302 directly across from cushion portion 206b such that an optical path is formed between the emitter unit 210 (not shown) housed within cushion portion 206a and the detector unit 212 (not shown) housed within cushion portion 206b.

The nasal cavity contains terminal branches of the internal carotid artery, namely, the anterior ethmoidal artery. Branches of the internal carotid artery, including the anterior ethmoidal artery, are among the last locations of the human body to experience vasoconstriction under physiological stress conditions because the internal carotid artery supplies blood to the brain. Furthermore, copious amounts of blood are supplied to the nasal cavity in order to warm incoming air before it reaches the lungs. Thus, blood flow through the anterior ethmoidal artery is not subject to substantial ANS regulation, making it an advantageous location from which to measure physiological parameters. The anterior ethmoidal artery 306 branches from the ophthalmic artery 308, which stems from the internal carotid artery 310. As shown in FIG. 3A, probe 200 is configured (e.g., shaped) such that cushion portion 206b is positioned over the anterior ethmoidal artery 306 such that detector unit 212 (not shown) receives light transmitted from emitter unit 210 (not shown) after passing through the anterior ethmoidal artery 306, where it may be absorbed by blood contained therein. Thus, probe 200 is positioned to obtain PPG signal data from blood originating from the internal carotid artery 310.

Turning now to FIG. 4, a partial front view of a face of a subject wearing a first embodiment of a nasal pulse oximetry probe 400 is shown. The center of the root of the nasal bridge is indicated at 402. The exact location of the center of the root of the nasal bridge 402 may vary for each patient. In the example of FIG. 4, the center of the root of the nasal bridge 402 is level with the bottom of the eye, as indicated by dashed line 404. However, in other examples, the center of the root of the nasal bridge 402 may be higher than dashed line 404 (e.g., more toward the eyebrows) or lower than dashed line 404 (e.g., more toward the lips). For example, the center of the root of the nasal bridge may lie along a central axis of the eyes (e.g., an axis that passes through the caruncula lacrimalis of each eye).

Bridge portion 408 of the nasal pulse oximetry probe (e.g., probe 200 of FIG. 2A) is shaped to be centered on the center of the root of the nasal bridge 402. In one example, the probe may include visual markers that assist an operator in positioning the probe in the proper position to accurately sense blood flow through the anterior ethmoidal artery. For example, a line may extend horizontally across the bridge portion of the probe, and the operator may position the probe such that the line is substantially aligned with a designated axis of the patient, e.g., the central axis of the eyes.

Cushion portions 406a and 406b are affixed to each side of the nose, for example, by an attachment mechanism such as adhesive. As described above with reference to FIG. 3A, emitter and detector units housed within respective cushion portions, and the cushion portions are shaped to position the emitter and detector units on opposite sides of the nose of the patient at a root of a nasal bridge when the optical probe is worn by the patient. The respective cushion portions are aligned such that an optical path is formed between them. The probe may be positioned with electrical wirings 418 running along the bridge of the nose and onto the forehead in order to minimize patient disturbance by the electrical wirings 418, which may otherwise obstruct the eyes, nose, or mouth, for example. However, the orientation of the electrical wirings does not impact the function of the probe and as such may have different configurations without departing from the scope of this disclosure.

Turning now to FIG. 5, a partial front view of a face of a subject wearing a second embodiment of a nasal pulse oximetry probe 500 is shown. Like components of FIGS. 4 and 5 are numbered similarly (e.g., 408 corresponds to 508). Whereas the nasal pulse oximetry probe of FIG. 4 may be attached to the patient with adhesive, the probe of FIG. 5 additionally or alternatively includes an attachment mechanism in the form of an eyeglass-type frame 510, which rests on ears 516 of the patient. The size and shape of frame 510 may vary. For example, frame 510 may be manufactured at different sizes and configurations to accommodate different types of patients (e.g., neonates, infants, pediatrics, and adults). In particular, the width of frame 510 may vary to accommodate patients with different head widths. The mechanisms for adjusting the probe widths may be part of the frame, which makes one frame suitable for broad range of head widths. Regardless of the size or shape of the frame 510, the frame 510 is configured (e.g., shaped) to position the emitter and detector units on opposite sides of the nose of the patient at a root of a nasal bridge when the optical probe is worn by the patient.

As described with reference to FIG. 4, bridge portion 508 of the nasal pulse oximetry probe is again shaped to be centered on the center of the root of the nasal bridge, indicated at 502, which is again level with the bottom of the eyes, as indicated by dashed line 504. Cushion portions 506a and 506b are pressed against sides of the nose by cushion holders 512a and 512b, respectively. Cushion holders 512a and 512b are attached to frame 510 by wires 514, which may be comprised of a flexible and/or malleable material, such as metals, polymers, polymer blends, etc., to enable the cushion holders to be adjusted with respect to the anatomy of the patient. Cushion holders 512a and 512b may apply pressure to the nasal bridge, which may push surface blood away from the skin of the nasal bridge. Further, the cushion holders may be shaped and sized to position the emitter and detector units at a designated position with respect to the nose e.g., across the root of the nasal bridge. Thus, the probe may more accurately measure light absorption by blood contained within the anterior ethmoidal artery, which is an internal structure, with less interference from surface blood in skin capillaries, for example.

As shown in FIG. 5, electrical wirings 518 again go up the bridge of the nose and onto the forehead of the patient. However, in another example, electrical wirings 518 are housed within frame 510. Furthermore, frame 510 may further house a control and processing unit (e.g., control and processing unit 107 of FIG. 1) for calculating physiological parameters from the signals received from the probe and a radio unit for communicating the physiological parameters to a display device (e.g., display unit 114 of FIG. 1).

The amount of light transmitted through a tissue varies according to changes in blood volume at the site. Specifically, the amount of light transmitted through vascular bed decreases during systole (e.g., more light is absorbed) and increases during diastole, resulting in periodic PPG waveforms (e.g., the AC/DC component). Multiple physiological parameters can be extracted from the PPG waveforms measured by a pulse oximetry probe. As an advantage of the copious blood supply to the nasal cavity and flowing through the anterior ethmoidal artery, the resulting PPG data measured by a nasal pulse oximetry probe has a high signal-to-noise ratio. For example, noise may be caused by non-physiological processes such as ambient light and bodily movement, which may be filtered out from the nasal PPG, because these artifact signals remain, in most cases, weak in comparison to the cardiac pulsation.

Turning now to FIG. 6, a high-level flow chart of a method 600 for calculating physiological parameters from the PPG signal is shown. Method 600 and the other methods described herein may be performed with a pulse oximetry system, such as pulse oximetry system 10 shown in FIG. 1. More specifically, method 600 and the other methods described herein may be executed by a control and processing unit of the pulse oximetry system (such as control and processing unit 107 shown in FIG. 1) according to instructions stored on a non-transitory memory of the system (e.g., memory 109 shown in FIG. 1) in combination with the various signals received at the control and processing unit from the system components and actuation signals sent from the system controller to the display device (e.g., display device 114 of FIG. 1). The methods included herein will be described with regard to a nasal pulse oximetry probe, although it should be understood that pulse oximetry probes placed on other parts of a body could be used without departing from the scope of the methods.

Method 600 begins at 602 and includes receiving probe output from a nasal pulse oximetry probe (e.g., probe 200 of FIG. 2A). As indicated at 604 and described with reference to FIG. 3A, the probe is positioned to measure blood within the anterior ethmoidal artery of a patient. In some examples, a clinician or other user may position the probe based on visual markers present on the probe in order for the probe to measure blood within the anterior ethmoidal artery. Additionally or alternatively, the clinician may scan the positions along the bridge of nose and find a position providing the strongest pulse.

At 606, method 600 includes calculating one or more physiological parameters from the probe output. For example, physiological parameters may include $SpO_2$, pulse transit time (PTT), blood circulation at the measurement site, respiration rate, and an indication of overall blood volume. Calculating the physiological parameters may include using algorithms stored in a memory of a control and processing unit (e.g., memory 109 of FIG. 1), including an $SpO_2$ algorithm (e.g., $SpO_2$ algorithm 111 of FIG. 1), a PTT algorithm (e.g., PTT algorithm 112 of FIG. 1), a respiration rate algorithm (e.g., respiration rate algorithm 116 of FIG. 1), a blood circulation algorithm (e.g., blood circulation algorithm 11 of FIG. 1), and a hypovolemia algorithm (e.g., hypovolemia algorithm 115 of FIG. 1). Examples of different calculations will be described with regard to FIGS. 7-11.

At 608, method 600 includes storing the physiological parameters (e.g., as calculated at 606) in the memory and/or displaying the parameters via a display device (e.g., display unit 114 of FIG. 1). Physiological parameters may be stored in the memory in order to monitor how the physiological parameters of the patient change over time. For example, if the patient is undergoing surgery, it may be beneficial to monitor for hypovolemia (decreased circulating blood volume) due to bleeding. Furthermore, the physiological parameters may be displayed on the display device so that a care provider may choose an appropriate course of action or make a diagnosis, for example. Following 608, method 600 ends.

FIG. 7 shows an example method 700 for calculating $SpO_2$ of a patient based on the PPG signal data obtained by a nasal pulse oximetry probe. Method 700 begins at 702 and includes determining the ratio of ratios

$$R = \frac{\left(\frac{AC}{DC}\right)_{red}}{\left(\frac{AC}{DC}\right)_{IR}}$$

, where AC is the valley-to-peak amplitude and DC is the baseline of the light transmission, based on probe output from the nasal pulse oximetry probe. Thus, to determine R, PPG waveforms for two wavelengths of light, one red and one infrared (IR), are recorded. At the red wavelength, Hb absorbs more light

and $HbO_2$ transmits more light. At the IR wavelength, $HbO_2$ absorbs more light and Hb transmits more light. Thus, the light transmission is higher and the PPG amplitude is correspondingly smaller at the red wavelength (and vice versa at the IR wavelength) when blood has a high oxygen saturation, and light transmission is lower and PPG amplitude larger at the red wavelength (and vice versa at the IR wavelength) when blood has a low oxygen saturation. As can be seen from the above equation for R, a low R value corresponds to a high amount of $HbO_2$ compared to Hb and thus a high arterial oxygen saturation.

At 704, method 700 includes calculating $SpO_2$ using empirical calibration. That is, first, the relationship between arterial oxygen saturation directly measured from an arterial blood sample and R, as calculated at 702, is determined in a volunteer test in laboratory setup. The pulse oximetry calibration is then established so that the $SpO_2$ output at each saturation level is set to show the blood oxygen saturation as closely as possible. The calibration is stored in the pulse oximeter memory, for example, in form of a lookup table or a polynomial function with R as input and $SpO_2$ as output.

At 706, method 700 includes outputting $SpO_2$. For example, $SpO_2$ may be displayed on a display device of the pulse oximeter (e.g., display device 114 of FIG. 1) and/or saved to a memory of the pulse oximeter (e.g., memory 109 of FIG. 1). Following 706, method 700 ends.

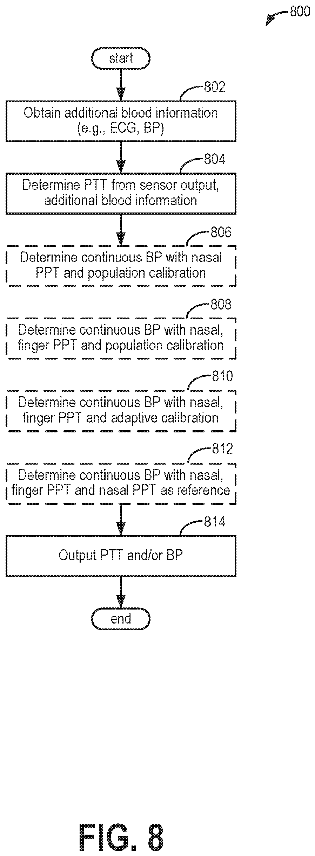

Turning now to FIG. 8, a method 800 is illustrated for calculating pulse transit time (PTT) of a patient from PPG signal data obtained by a nasal pulse oximetry probe (e.g., probe 200 of FIG. 2A) positioned to measure blood originating from the internal carotid artery. PTT is defined as the time it takes a pulse pressure waveform to propagate through a length of the arterial tree, typically from the aorta to a peripheral arterial site. PTT is usually measured, in part, with information from an electrocardiogram (ECG). As such, a first time point is the ECG R-peak time, and a second time point is the beginning of the systolic increase of blood volume, e.g., when the PPG waveform begins to rise, at the peripheral arterial site, typically the measurement site on which the PPG probe is positioned (e.g., the nasal bridge or finger). PTT can be related to blood pressure (BP), which thus enables continuous non-invasive BP measurement. Specifically, PTT can be used to determine a pulse wave velocity, which is related to vessel stiffness. The greater the stiffness, the higher the pulse wave velocity. Artery stiffness is modulated by factors such as age, atherosclerosis, and BP. At high BPs, artery walls are tense and hard, making the pulse wave travel faster (e.g., PTT is reduced). At low BPs, the artery walls have less tension, making the pulse wave travel slower (e.g., PTT is increased).

Refering to FIG. 8, method 800 for blood pressure determination begins at 802 and includes measuring electrical activity of the a patient's heart over a period of time using electrodes placed on the chest. The resulting data may be output as an ECG, for example. Simultaneously, PPG signals are measured at the nasal and/or finger site (as described above with respect to FIG. 6).

At 804, method 800 includes determining a nasal PTT and/or a finger PTT from the ECG R-peak and a specific time point in the PPG systolic rise at the nasal and/or finger sites. For example, a control and processing unit (e.g., control and processing unit 107 of FIG. 1) may use a PTT algorithm (e.g., PTT algorithm 112 of FIG. 1), in which an ECG processing algorithm detects the ECG R-peak by detecting the maximum of the R-peak and a PPG processing algorithm detects specific time points during the systolic rise. For example, the points of maximum derivative (e.g., the time at which the first derivative of the PPG waveform is maximal) of the nasal and finger PPG waveforms may be detected. Therefore, two PTTs, one for the nasal PPG and the other for the finger PPG, are determined. The R-wave peak time corresponds to when the heart ventricle contracts, and the PPG maximum derivative time corresponds to when blood ejected from the heart by the ventricle contraction reaches the measurement site. Of note, there is a ~250 ms delay from the R-peak for the pulse wave to reach a finger. However, this delay does not occur at the nasal site because the distance from heart is shorter and because vasoconstriction in the artery due to ANS regulation is almost non-existent in the (high priority) intra-cranial blood flow. Therefore, the stiffness of the carotid artery, and thereby, the pulsewave velocity, is influenced only by blood pressure, whereas at peripheral sites, the pulse velocity is also affected by other (confounding) factors, such as sympathetical activation (vasoconstriction) or blood volume changes. Consequently, the PTT determined by a nasal pulse oximeter most likely reflects the effects of BP changes more profoundly than PTT determined by a pulse oximeter measuring blood at the finger or other extremity. On the other hand, because the distance of the finger from the heart is longer, the blood pressure changes the absolute PTT value measured at the finger more than at the nasal site.

The PTT(s) may be used in one or more algorithms to determine continuous blood pressure (BP). There are several ways to use PTT to measure BP. In a first example, as indicated at 806, continuous BP may be determined based on nasal PTT and a population calibration. As described above, the PTT is determined from the ECG R-peak to the systolic rise (maximum derivative) in the nasal PPG. As discussed above, the benefit of this method is that the PTT is least affected by confounding ANS regulation, hypovolemia, hypothermia, and atherosclerosis of peripheral arteries, for example. The BP may be measured by first determining the relationship between PTT and BP in a large patient population. In these calibration runs, the BP is determined, for example, invasively or using non-invasive blood pressure (NIBP) measurement, after which the functional relationship between BP and PTT may be established. This relationship may then be used on patients of the same population characteristics. For example, a calibration equation may be calculated that is different for different patient populations, such as children and adults.

In another example for a continuous BP measurement, as indicated at 808, the continuous BP may be determined based on the PTTs determined for both the nasal and finger sites and the population calibration described above. The PTTs may be calculated using the ECG R-peak time point as a reference. A benefit of this embodiment is that a BP change is larger in absolute value for the finger PTT. As discussed above, however, the finger PTT may be confounded by ANS regulation or other reasons. In this situation, the nasal PTT may be beneficially used to individualize the relationship between the two measured PTTs and the patient's individual BP. As a result, a large part of the patient-to-patient variability in the particular patient population may be removed.

As a further example, as indicated at 810, continuous blood pressure may be determined based on the finger and nasal PTTs and an adaptive calibration. For example, a two point calibration of the measured PTT values against a NIBP measurement at two different levels of BP may be calculated for one patient. The PTT and BP values may be obtained close in time so that confounding factors are likely constant during the two-point calibration interval. The assumption is valid for such confounding factors as hypovolemia, hypothermia, and blood vessel antheroclerosis. In one example, a linear model between BP and the nasal and finger PTT (PTT1 and PTT2, respectively) may be assumed, according to equation A:

$$\begin{pmatrix} BP \\ C \end{pmatrix} = \begin{pmatrix} c11 & c12 \\ c21 & c22 \end{pmatrix} \begin{pmatrix} PTT1 \\ PTT2 \end{pmatrix}$$

The above equation has four unknown coefficients, c11, c12, c21, and c22. In a first step of calibration, the coefficients c11 and c12 are determined so that the correct BP values are obtained at the two NIBP levels. The output parameter C may be used for detecting changed confounding factor situations, e.g., those that may occur due to blood volume changes or temperature changes. At normal patient conditions, the value of C may be set to be a fixed constant at both BP calibration levels. The coefficients c21 and c22 may then be determined in the calibration phase at the two levels of BP. Multiple point calibration may be used to improve the calibration process. In this case, the coefficients c11, c12, c21, and c22 are determined using a statistical optimization analysis, such as linear least square optimization. After calibration, the equation Eq. A is used to calculate BP from the measured PTT values, e.g., PTT2 for the finger and PTT1 for the nasal site. In one example, the constant C may be used to alert about a changed patient condition due to reasons other than BP. When a change in C is observed, the coefficients c11 and c22 may be determined again by requesting NIBP calibration from a user. The NIBP measurement is activated at least at one time point and at one BP level, and the corrections for the coefficients c11 and c12 (as well as c21 and c22) are determined.

The above adaptive method may be improved by using a multiple point original calibration with a non-linear relationship between PTT1 and PTT2 and a large patient population with different (mixed) levels of confounding factors. For example, BP may be calculated as BP=f(PTT1, PTT2, + other variables such as HR), in which f is the non-linear function for BP having PTTs and other physiological parameters as independent variables.

A further example for continuous BP measurement using PTT measurement is indicated at 812, where the continuous BP is determined based on the finger and nasal PTTs using the nasal PTT as a reference (rather than the ECG R-peak as described above). Since PTT to the nasal root is very short (e.g., relative to the finger PTT) at some tens of milliseconds, the nasal plethysmogram may be used as a reference time point for the finger PTT. In this case, the ECG waveform is not needed. The finger PTT is then calculated from the nasal pleth systolic rise to the finger pleth systolic rise, for example, both determined as the maximum derivative of the pleth waveform. This PTT may then be calibrated against NIBP BP determinations using standard methods.

At 814, method 800 includes outputting PTT and/or BP. For example, PTT and/or BP may be displayed on a display device of the pulse oximeter (e.g., display device 114 of FIG. 1) and/or saved to a memory of the pulse oximeter (e.g., memory 109 of FIG. 1).

Following 814, method 800 ends.

Turning now to FIG. 9, a method 900 for determining respiration rate using a nasal pulse oximetry probe (e.g., probe 200 of FIG. 2A) positioned to measured blood originating from the internal carotid artery is shown. As a patient breathes, changes in intrathoracic pressure throughout a respiratory cycle cause respiration-related variations in the PPG waveform, including baseline modulation (the DC component, due to changes in venous return), amplitude modulation (the AC component, due to a decreased left ventricular stroke volume during inspiration), and frequency modulation (due to the fact that heart rate increases during inspiration and decreases during expiration). In standard pulse oximetry, these variations are often filtered out for determining $SpO_2$. However, the waveform modulations may be used to determine the respiration rate of a subject from the PPG signal. Notably, respiration-related variation is stronger in PPG signals measured at the root of the nasal bridge than measured at a finger or ear, resulting in better signal-to-noise separation, which may improve the accuracy of respiration rate calculations. The inventors herein have recognized that another parameter, $SpO_2$, experiences respiration-related variation. Microfluctuations in

$SpO_2$ are not detectible in the PPG signal measured at a finger, for example, but are detectible in the PPG signal measured at the root of the nasal bridge. Thus, the microfluctuations in $SpO_2$ measured by a nasal pulse oximeter may also be used to calculate respiration rate, as described below.

Method 900 begins at 902 and includes calculating a first respiration rate based on probe output in a time domain. PPG data is collected over time, resulting in the PPG waveform. A control and processing unit (such as control and processing unit 107 of FIG. 1) may extract respiration-related variations in the PPG waveform and use an algorithm (e.g., respiration rate algorithm 116 of FIG. 1) to calculate respiration rate based on these variations. For example, the control and processing unit may extract one or more of the baseline modulation, amplitude modulation, and frequency modulation of the PPG waveform to calculate the respiration rate. Respiration rate may be calculated from probe data obtained over a first predetermined duration (e.g., 45 seconds), for example. Further, the respiration rate may be recalculated at a predetermined frequency (e.g., every 5 seconds).

At 904, method 900 includes calculating a second respiration rate based on microfluctuations in $SpO_2$. In the previous example, data from a PPG waveform measured at one wavelength (typically infrared) may be used to determine respiration rate. In another example, the complete two waveform data, e.g., the red and infrared PPGs, may be used to determine respiration rate. The respiration rate may be determined from the ratio of ratios, R, by first calculating R separately for systolic and diastolic phases of the two PPGs, with R calculations repeated pulse by pulse (e.g., for each heartbeat), as described further below with reference to FIG. 13.

Turning briefly to FIG. 12, an example graph 1200 illustrating how R may be determined separately for systole and diastole is shown. As shown, R may be determined by plotting $(AC/DC)_{red}$ (Y-axis) against $(AC/DC)_{IR}$ (X-axis), with the resulting graph forming a loop. A top portion of the loop, indicated at 1202, comprises measurements taken during systole (the phase of plethymogram during which the signal increases), and a bottom portion of the loop, indicated at 1204, comprises measurements taken during diastole (the phase of the plethysmogram during which the signal decreases). The slope of the top of the loop 1202 is equal to R during systole ($R_{systole}$), while the slope of the bottom of the loop 1204 is equal to R during diastole ($R_{diastole}$). A difference between $R_{systole}$ and $R_{diastole}$ is due to respiration-related variation.

Continuing to FIG. 13, an example graph 1300 is shown illustrating respiration rate (plot 1302), wherein a ratio of ratio of ratios (Y-axis), defined as the ratio of $R_{systole}$ and $R_{diastole}$, is plotted versus heartbeat (X-axis). The ratio of ratio of ratios gives the respiration signal, which may be used to determine the second respiration rate using a respiration rate algorithm (e.g., respiration rate algorithm 116 of FIG. 1).

Returning to FIG. 9, at 906, method 900 includes outputting a calculated respiration rate based on one or more of the first respiration rate and the second respiration rate. In one example, the first and the second respiration rates are averaged and output as the calculated respiration rate. In another example, one respiration rate (either the first or the second) is output responsive to the magnitude of the difference between the first and second respiration rate being within a threshold. Following 906, method 900 ends.

By calculating the respiration rate in more than one way, the accuracy of the resulting value may be improved compared with calculating the respiration rate one way. Further, the respiration rate may be more accurately determined using a nasal pulse oximetry probe positioned at the root of the nasal bridge compared with a pulse oximetry probe positioned at an extremity due to stronger respiration-related variation observed at the nasal site.

Changes in respiration-related variation can be used to determine additional physiological parameters. Turning now to FIG. 10, a method 1000 for determining if a patient is hypovolemic using a nasal pulse oximetry probe (e.g., probe 200 of FIG. 2A) positioned to measured blood originating from the internal carotid artery is shown. Hypovolemia is defined as a state of decreased blood volume, which may be caused by blood loss or sodium depletion, for example.

Method 1000 begins at 1002 and includes transforming probe output to a frequency domain. For example, the probe output obtained over a duration (e.g., 60 seconds) may be analyzed in the frequency domain using a fast Fourier transform (FFT). The resulting spectrum shows the PPG signal plotted as amplitude density against frequency.

Turning briefly to FIG. 14, a graph 1400 of example spectra of transformed PPG signal data is shown. The X-axis of graph 1400 represents frequency, and the Y-axis represents amplitude density. An example spectrum of transformed PPG signal data measured from a normovolemic patient is represented by a dashed line, and an example spectrum of transformed PPG signal data measured from a hypovolemic patient is represented by a solid line. A first peak indicated at 1402a (normovolemia) and 1402b (hypovolemia) occurs in each spectrum at a respiratory frequency (~10/min), and a second peak indicated at 1404a (normovolemia) and 1404b (hypovolemia) occurs in each spectrum at a cardiac frequency (which typically ranges from 40-90/min for a resting adult). Each peak also has corresponding higher frequency harmonics.

During hypovolemia, there is an increase in respiratory-induced variation due to the greater impact positive pressure has on a lower blood volume. Therefore, at the respiratory frequency, the first peak 1402b of the spectrum corresponding to the hypovolemic patient (solid line) has a greater amplitude density than the first peak 1402a of the spectrum corresponding to the normovolemic patient (dashed line). Thus, it may be possible to determine hypovolemia in a patient based on the amplitude density at the respiratory frequency (e.g., the respiration-related variation), as described below. In an embodiment, the ratio of the spectral amplitudes at the respiratory frequency and cardiac frequency is determined. This ratio is high for a hypovolemic patient and low for a normo- or hypervolemic patient. Of note, the respiratory frequency peak of transformed PPG signal data acquired with a finger pulse oximeter is weaker and less sensitive to volemia status than the respiratory frequency peak of transformed PPG signal data acquired with a nasal pulse oximeter. Thus, it may be advantageous to determine volemia status with the nasal pulse oximeter.

Returning to FIG. 10, at 1004, method 1000 includes determining the amplitude density of the respiratory frequency signal (e.g., peaks 1402a and 1402b in FIG. 14). The amplitude density of the respiratory frequency signal gives an indication of the respiration-related variation.

At 1006, the method includes indicating hypovolemia when the amplitude density is greater than a threshold. In another example, hypovolemia is indicated when the amplitude density of the respiratory frequency signal increases by a threshold amount. In still another example, hypovolemia is indicated when the ratio of the respiratory and cardiac spectral peaks is greater than a threshold. An indication of hypovolemia may be output on a display device (e.g., display device 114 of FIG. 1), for example. Following 1006, method 1000 ends.

FIG. 11 shows a method 1100 for determining blood circulation at a measurement site (e.g., the root of the nasal bridge) using a nasal pulse oximetry probe (e.g., probe 200 of FIG. 2A). Blood circulation may be determined by changing the volume of venous blood returned to the heart, as described below. The change in the blood volume returned to the heart may be determined in various ways, for example, by performing a passive leg raising procedure, changing the patient position (supine, sitting up-right positioning), or by changing intrathoraxic pressure, if the patient is on a ventilator. As will be described in more detail below, the change in blood volume returned may be determined using a change in positive end-expiratory pressure (PEEP). Because the measurement site is supplied by the internal carotid artery, blood circulation at the measurement site also gives an indication of blood flow to the brain. Such an indication cannot be obtained by measuring blood circulation at an extremity, such as a finger or ear.

Method 1100 begins at 1102 and includes obtaining a first probe output with a first amount of positive end-expiratory pressure (PEEP). The probe output may be the DC level of the total light transmission. It is further noted that only the infrared output may be used. PEEP refers to a pressure in the lungs that exists at the end of expiration. Extrinsic PEEP may be applied with a ventilator, and an amount of PEEP may be controlled by settings of the ventilator. PEEP may contribute to decreased venous return, which results in increased blood accumulation, as measured by the pulse oximetry probe at the measurement site.

At 1104, method 1100 includes obtaining a second probe output with a second amount of PEEP. For example, the second amount of PEEP may be higher than the first amount of PEEP, resulting in further blood accumulation. Alternatively, the second amount of PEEP may be less than the first amount of PEEP, resulting in reduced blood accumulation compared with the first amount of PEEP.

Turning briefly to FIG. 15, a graph 1500 is shown illustrating an example of PPG signal data acquired by a nasal pulse oximeter during different amounts of PEEP. The X-axis represents time, with time increasing from left to right, and the Y-axis represents the intensity of transmitted light, with intensity increasing from bottom to top. The red output is shown at plot 1502, and the infrared output is shown at plot 1504. During time ranges t1 and t3, a first, lower amount of PEEP is applied (e.g., 5 cmH$_2$O). During time range t2, a second, higher amount of PEEP is applied (e.g., 12 cmH$_2$O). During time range t2, the intensity of transmitted light in the red channel (plot 1502) decreases compared to time range t1 due to further blood accumulation, as the second, higher amount of PEEP further blocks venous return to the heart. There is also a small decrease in the intensity of transmitted light in the infrared channel (plot 1504) during time range t2. The relative changes depend on the overall (venous and arterial) saturation of blood at the site. During time range t3, the intensity of transmitted light in the red channel (plot 1502) increases compared to t2 as the amount of PEEP is reduced to the first, lower amount of PEEP.

Returning to FIG. 11, at 1106, method 1100 includes determining blood circulation based on a difference between the first probe output and the second probe output. For example, a control and processing unit (such as control and processing unit 107 of FIG. 1) in communication with the probe may use the difference between the first probe output (the DC level of the infrared PPG at the first amount of PEEP) and the second probe output (the DC level of the same infrared PPG at the second amount of PEEP) and an algorithm (e.g., blood circulation algorithm 110 of FIG. 1) to determine the blood circulation. Whereas blood flow in the extremities is subject to regulation by the ANS (such as vasoconstriction), which may block the effects of PEEP, blood flow at the root of the nasal bridge is not. As such, the blood circulation algorithm for the nasal pulse oximeter may include less filtering than, for example, a blood circulation algorithm for a finger pulse oximeter. Further, the control and processing unit may use the first probe output and the second probe output to determine a difference in respiration-related variation for the first amount of PEEP and the second amount of PEEP. For example, the difference of the DC levels at the nasal root (or other blood volume indicator signals, such as PPG pulse amplitude variations at the nasal root) at the two different amounts of PEEP may be used to create new parameters that show hypovolemia. These may be used to create more reliable measures of hypovolemia than is possible at one amount of PEEP. Following 1106, method 1100 ends.

Thus, the systems and methods described herein provide for determining various physiological parameters of a patient, including $SpO_2$, pulse transit time, respiration rate, hypovolemia, and intracranial blood circulation, from an output of a nasal pulse oximetry probe configured to measure blood flowing through the anterior ethmoidal artery. In some examples, in addition to determining the physiological parameters using the output from the nasal pulse oximetry probe, an output from a second pulse oximetry probe located at an extremity of the patient, such as a finger or ear, may also be used. Physiological parameters calculated from the output of the nasal pulse oximetry probe may be compared to physiological parameters calculated from the output of the second pulse oximetry probe to determine a state of the autonomic nervous system, for example. For example, if a pulse wave is detected at the nasal site but not at the extremity (or if the pulse wave is diminished at the extremity), it may be determined that the ANS is regulating blood flow to the extremities. Such a comparison is enabled by the fact that blood vessels in the extremities are subject to vasoconstriction and the anterior ethmoidal artery is not. As a result, the physiological parameter calculations described herein (e.g., with respect to FIGS. 6-11) may be performed accurately even when a patient is undergoing vasoconstriction, such as when the patient is hypothermic. In another example, if a physiological parameter calculated from each probe output differs by more than a threshold, the physiological parameter may be re-measured. In still another example, the calculations from each probe output may be averaged.

The technical effect of measuring the blood oxygen saturation using a nasal pulse oximetry probe is a more accurate measurement that is not confounded by vasoconstriction.

An example provides a system for an optical probe, comprising a light emitter and a light detector each coupled to a substrate, the light emitter and light detector configured to measure blood originating from an internal carotid artery of a patient; and an attachment mechanism configured to couple the optical probe to a nose of the patient, the light emitter and light detector positioned to be on opposite sides of the nose of the patient at a root of a nasal bridge when the optical probe is worn by the patient. In examples, the optical probe is configured to measure arterial oxygen saturation at the root of the nasal bridge.

The optical probe may further comprise a first soft cushion-like structure that encompasses the light emitter and a second soft cushion-like structure that encompasses the light detector, each cushion-like structure conforming to the nose of the patient, wherein each cushion-like structure is deformable. In an example, each cushion-like structure includes deformable gel. In one example, the first cushion-like structure covers the light detector and the second cushion-like structure covers the light emitter, each cushion-like structure being at least partially transparent such that light from the light emitter is configured to pass through the first cushion-like structure and through the second cushion-like structure to the light detector.

The attachment mechanism may include a pressure element configured to block surface blood circulation of skin of the patient at the root of the nasal bridge. In examples, the attachment mechanism is an eyeglass-type frame.

The light emitter may include a light emitting diode (LED) configured to emit light having a wavelength of between 760 and 950 nm. In an example, the light emitter includes a first LED having an emission wavelength in a range between 620 and 690 nm and a second LED having an emission wavelength in a range between 760 nm and 950 nm.

The substrate may comprise a T-shape with opposite T-ends of the substrate housing the light detector and light emitter, respectively, and a T-foot of the substrate carrying electrical wirings from the optical probe to a measurement unit along the nasal bridge of the nose.

The optical probe may further comprise a control and processing unit that processes signals from the light detector and calculates physiological parameters, the control and processing unit communicatively coupled to a display unit. In an example wherein the attachment mechanism is an eyeglass-type frame, and the control and processing unit may be coupled to the eyeglass-type frame.

Another example system for an optical probe comprises a light emitter and a light detector shaped to be attached on a nasal root of a patient and configured to measure light transmission through blood originating from an internal carotid artery of the patient and output a light transmission signal; and a control and processing unit including instructions to extract a respiration-related variation from a photoplethysmogram obtained from the light transmission signal. The control and processing unit may be configured to extract a respiration rate and/or an amplitude of the respiration-related variation. Futher, the control and processing unit may be configured to detect hypovolemia from the respiration-related variation. Futher still, the control and processing unit may be configured to measure the respiration-related variation at two physiological conditions that reflect different venous blood return volumes to a heart of the patient, for which a difference of the respiration-related variation is determined.

Another example of a system for an optical probe comprises a light emitter and a light detector shaped to be attached on a nasal root of a patient and configured to measure light transmission through blood originating from an internal carotid artery of the patient and output a light transmission signal; and a control and processing unit having instructions stored in memory or hardware configured to determine microfluctuations in arterial oxygen saturation based on the light transmission signal obtained over a duration. The control and processing unit may be configured to perform a first arterial oxygen saturation calculation for systole and a second arterial oxygen saturation calculation for diastole and determine the microfluctuations in arterial oxygen saturation based on a difference of the first and second arterial oxygen saturation calculations. In an example, the control and processing unit may be configured to use the microfluctuations in arterial oxygen saturation to calculate a respiration rate of the patient. In another example, the control and processing unit may be configured to use the microfluctuations in arterial oxygen saturation to determine a respiration-related variation.

In another representation, a method comprises receiving probe output from a nasal pulse oximetry probe attached to a subject at a measurement site and calculating one or more physiological parameters from the probe output. In examples, the measurement site is a root of a nasal bridge. In one example, calculating the one or more physiological parameters includes calculating $SpO_2$. In examples, $SpO_2$ may be calculated from a ratio of ratios (R), wherein the ratio of ratios is determined from the probe output measured at two different wavelengths of light. In one example, $SpO_2$ may be calculated using R and an empirical calibration that relates arterial oxygen saturation values directly measured from arterial blood samples and corresponding R values.

In another example, calculating the one or more physiological parameters includes calculating pulse transit time (PTT). For example, calculating PTT may comprise determining a first time from an electrocardiogram (ECG), wherein the first time corresponds to an R-peak time, and a second time from the probe output, wherein the second time corresponds to a time at which blood volume increases at the measurement site. Further, in some examples, PTT may used to calculate continuous blood pressure (BP). For example, continuous BP may be calculated using PTT and a population calibration, wherein the population calibration includes measuring PTT and measuring BP for a plurality of subjects and determining a functional relationship between BP and PTT. In another example, continuous BP may be calculated using PTT and an adaptive calibration. The adaptive calibration may include measuring PTT and BP at two or more BP levels and determining a relationship between BP and PTT.

In an example, calculating the one or more physiological parameters includes calculating blood circulation at the measurement site. In examples, calculating blood circulation comprises obtaining a first probe output at a first amount of positive end-expiratory pressure (PEEP) and obtaining a second probe output at a second amount of PEEP. In one example, calculating blood circulation further comprises determining a difference in respiration-related variation for the first amount of PEEP and the second amount of PEEP from the first probe output and the second probe output, respectively, wherein the difference in respiration-related variation gives an indication of total blood volume.

In another example, calculating the one or more physiological parameters includes calculating respiration rate. In one example, calculating respiration rate comprises obtaining probe output over a duration and identifying a variation in the probe output over the duration, wherein the frequency of the variation is used to calculate respiration rate. The variation in the probe output may include one or more of a baseline modulation, an amplitude modulation, and a frequency modulation. In another example, calculating respiration rate comprises calculating microfluctuations in $SpO_2$ for each heartbeat of the subject, wherein the microfluctuations in $SpO_2$ are determined by calculating a ratio of a first ratio of ratios (R) for a systolic phase and a second R for a diastolic phase of each heartbeat.

In examples, calculating the one or more physiological parameters includes calculating an indication of overall blood volume. In examples, calculating the indication of overall blood volume comprises transforming the probe output to a frequency domain, determining an amplitude density at a respiratory frequency, and determining an amplitude density at a cardiac frequency. In one example, the overall blood volume is indicated to be low responsive to the amplitude density at the respiratory frequency being greater than a first threshold. In another example, calculating the indication of overall blood volume further comprises determining a ratio of the amplitude density at the respiratory frequency and the amplitude density at the cardiac frequency, wherein the overall blood volume is indicated to be low if the ratio is greater than a second threshold.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc., are used merely as labels and are not intended to impose numerical requirements or a particular positional order on their objects.

**Claims**

**1.** A system (10) comprising:

an optical probe (11; 200) shaped to be attached on a nasal root of a patient, wherein the optical probe comprises a light emitter (100) and a light detector (103) configured to measure light transmission through blood originating from an internal carotid artery of the patient and output a light transmission signal; and

a control and processing unit (107) having instructions stored in memory (109) or hardware configured to determine a respiration rate of the patient based on the light transmission signal obtained over a duration,

wherein the control and processing unit is configured to perform a first arterial oxygen saturation calculation for systole and a second arterial oxygen saturation calculation for diastole, and

wherein the control and processing unit is further configured to determine the respiration rate using a ratio of a result of the first arterial oxygen saturation calculation and a result of the second arterial oxygen saturation calculation.

**Patentansprüche**

1. System (10), das Folgendes umfasst:

eine optische Sonde (11; 200), die so geformt ist, dass sie an einer Nasenwurzel eines Patienten angebracht werden kann, wobei die optische Sonde einen Lichtemitter (100) und einen Lichtdetektor (103) umfasst, ausgelegt zum Messen der Lichttransmission durch Blut, das von einer internen Arteria carotis des Patienten stammt, und Ausgeben eines Lichttransmissionssignals; und

eine Steuer- und Verarbeitungseinheit (107) mit in einem Speicher (109) gespeicherten Anweisungen oder Hardware, ausgelegt zum Bestimmen einer Atemfrequenz des Patienten basierend auf dem über einen Zeitraum erhaltenen Lichttransmissionssignal,

wobei die Steuer- und Verarbeitungseinheit dazu ausgelegt ist, eine erste Berechnung der arteriellen Sauerstoffsättigung für die Systole und eine zweite Berechnung der arteriellen Sauerstoffsättigung für die Diastole durchzuführen, und

wobei die Steuer- und Verarbeitungseinheit ferner dazu ausgelegt ist, die Atemfrequenz unter Verwendung eines Verhältnisses eines Ergebnisses der ersten Berechnung der arteriellen Sauerstoffsättigung und eines Ergebnisses der zweiten Berechnung der arteriellen Sauerstoffsättigung zu bestimmen.

**Revendications**

1. Système (10) comprenant :

une sonde optique (11 ; 200) mise en forme pour être fixée sur une racine de nez d'un patient, la sonde optique comprenant un émetteur de lumière (100) et un détecteur de lumière (103) configurés pour mesurer une transmission de lumière via le sang provenant d'une artère carotide interne du patient et délivrer un signal d'émission de lumière ; et

une unité de commande et de traitement (107) ayant des instructions stockées en mémoire (109) ou du matériel configuré pour déterminer une fréquence respiratoire du patient sur la base du signal d'émission de lumière obtenu sur une durée,

l'unité de commande et de traitement étant configurée pour réaliser un premier calcul de saturation du sang artériel en oxygène pour une systole et un deuxième calcul de saturation du sang artériel en oxygène pour une diastole, et

l'unité de commande et de traitement étant en outre configurée pour déterminer la fréquence de respiration en utilisant un rapport d'un résultat du premier calcul de saturation du sang artériel en oxygène et un résultat du deuxième calcul de saturation du sang artériel en oxygène.

**FIG. 1**

**FIG. 2A**

**FIG. 2B**

TO INNER/CENTRAL BRAIN

FIG. 3A

FIG. 3B

FIG. 4

FIG. 5

FIG. 6

FIG. 7

800

start

↓ 802

Obtain additional blood information
(e.g., ECG, BP)

↓ 804

Determine PTT from sensor output,
additional blood information

↓ 806

Determine continuous BP with nasal
PPT and population calibration

808

Determine continuous BP with nasal,
finger PPT and population calibration

810

Determine continuous BP with nasal,
finger PPT and adaptive calibration

812

Determine continuous BP with nasal,
finger PPT and nasal PPT as reference

↓ 814

Output PTT and/or BP

↓

end

# FIG. 8

```
                                      ┌─900
          ╭─────────╮
          │  start  │
          ╰─────────╯
               │              ┌─902
               ▼
     ┌──────────────────────┐
     │ Calculate first      │
     │ respiration rate     │
     │ based on sensor      │
     │ output in time domain│
     └──────────────────────┘
               │              ┌─904
               ▼
     ┌──────────────────────┐
     │ Calculate second     │
     │ respiration rate     │
     │ based on             │
     │ microfluctuations    │
     │ in SpO₂              │
     └──────────────────────┘
               │              ┌─905
               ▼
     ┌──────────────────────┐
     │ Output calculated    │
     │ respiration rate     │
     │ based on one or more │
     │ of first respiration │
     │ rate and second      │
     │ respiration rate     │
     └──────────────────────┘
               │
               ▼
          ╭─────────╮
          │   end   │
          ╰─────────╯
```

**Calculate first respiration rate based on sensor output in time domain** — 902

**Calculate second respiration rate based on microfluctuations in $SpO_2$** — 904

**Output calculated respiration rate based on one or more of first respiration rate and second respiration rate** — 905

## FIG. 9

— 1000

start

**Transform sensor output to frequency domain** — 1002

**Determine amplitude density of respiratory frequency signal** — 1004

**Indicate hypovolemia when amplitude density is greater than threshold** — 1006

end

## FIG. 10

— 1100

start

**Obtain first sensor output with first amount of positive end-expiratory pressure (PEEP)** — 1102

**Obtain second sensor output with second amount of PEEP** — 1104

**Determine blood circulation based on difference between first sensor output and second sensor output** — 1106

end

## FIG. 11

FIG. 12

FIG. 13

EP 3 570 738 B1

FIG. 14

FIG. 15

EP 3 570 738 B1

**REFERENCES CITED IN THE DESCRIPTION**

**Patent documents cited in the description**

- US 7648463 B1

- US 2015038810 A1

**Non-patent literature cited in the description**

- **N. CONSTANT ; O. DOUGLAS-PRAWL ; S. JOHNSON ; K. MANKODIYA.** Pulse-Glasses: An unobtrusive, wearable HR monitor with Internet-of-Things functionality. *2015 IEEE 12th International Conference on Wearable and Implantable Body Sensor Networks (BSN), Cambridge, MA, USA,* 2015, 1-5